# EUROPÄISCHE PATENTSCHRIFT

(11) **EP 0 422 523 B1**
(45) Veröffentlichungstag und Bekanntmachung des Hinweises auf die Patenterteilung: **15.03.1995**
(21) Anmeldenummer: 90119161.9
(22) Anmeldetag: 05.10.1990
(51) Int. Cl.: C07D 317/36, C07D 319/06

(54) **Verfahren zum Herstellen von cyclischen Kohlensäureestern**
Process for the preparation of cyclic carbonates
Procédé de préparation de carbonates cycliques

(30) Priorität: 07.10.1989 DE 3933617
(43) Veröffentlichungstag der Anmeldung: 17.04.1991
(73) Patentinhaber: BOEHRINGER INGELHEIM KG, 55216 Ingelheim (DE); BOEHRINGER INGELHEIM INTERNATIONAL GmbH, 55218 Ingelheim am Rhein (DE)
(72) Erfinder: Müller, Klaus Robert, Dr.-Dipl.-Ing. Dipl.-Chem., W-6507 Ingelheim am Rhein (DE); Buchholz, Berthold, Dr., W-6507 Ingelheim am Rhein (DE); Hess, Joachim, Dr.-Dipl.-Chem., W-6530 Bingen (DE)

(56) Entgegenhaltungen:
- US-A- 3 663 569

## Beschreibung

Die vorliegende Erfindung betrifft ein neues und industriell anwendbares Verfahren zur Herstellung von cyclischen Kohlensäureestern der allgemeinen Formel (I) 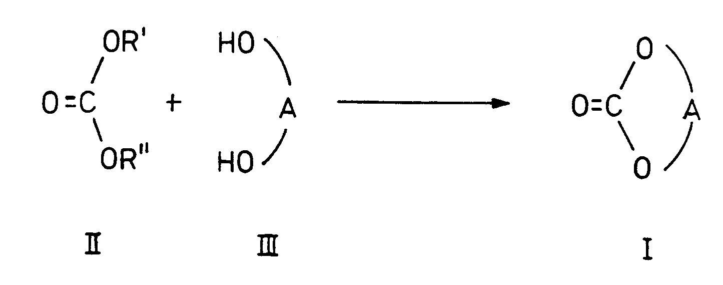
worin
A eine gegebenenfalls substituierte Alkylen-Brücke folgenden Typs
-CR¹R²-CH₂-, -CHR¹-CHR²-,
-CR¹R²-(CH₂)₂-, -CHR¹-CHR²-CH₂-,
-CH₂-CR¹R²-CH₂-, -CHR¹-CH₂-CHR²-,
und
- R¹ und R²: gleich oder verschieden sein können und Wasserstoff, Methyl, Ethyl, Propyl, Isopropyl, n-Butyl, sec-Butyl, tert.-Butyl, Allyl, But-2-enyl, But-3-enyl, Pent-2-enyl, Pent-3-enyl, Pent-4-enyl, Phenyl oder Benzyl
und
- R′ und R˝: Methyl, Ethyl Propyl oder Phenyl
bedeuten können.

Cyclische Kohlensäureester finden zum einen als Lösungsmittel - wie z.B. Ethylencarbonat (1,3-Dioxolan-2-on) - Verwendung, zum anderen verkörpern sie essentielle Bausteine von sog. bioabbaubaren Polymeren - wie z.B. Trimethylencarbonat (1,3-Dioxan-2-on) - die ihrerseits wiederum in einer Vielzahl von Anwendungsformen eingesetzt werden (chirurgisches Nahtmaterial, Gefäßimplantate, Vorrichtungen zur Osteosynthese etc.).

Der Einsatz als Lösungsmittel wie auch - im besonderen Maße - die Verwendung beim Aufbau von bioabbaubaren Polymeren stellen hohe Anforderungen an die Reinheit der jeweiligen Kohlensäureester.

So besteht beim Einsatz als Lösungsmittel die Gefahr, daß sich die im Solvenz befindlichen Verunreinigungen beim Entfernen des Lösungsmittels in dem ursprünglich gelösten Stoff anreichern.

Auf der anderen Seite werden die physikalischen und chemischen Eigenschaften der erwähnten Polymere - wie z.B. Zugfestigkeit, bei der Polymerisation erreichbare Molmasse und Abbaurate (Hydrolysegeschwindigkeit) - erheblich von der Reinheit der eingesetzten Monomere - beispielsweise Trimethylencarbonat - auf empfindliche Weise beeinflußt.

Unter dem Aspekt einer industriellen Herstellung der cyclischen Kohlensäureester ist es ferner wünschenswert, ein Syntheseverfahren zur Verfügung zu stellen, welches cyclische Carbonate liefert, die möglichst alle Reinheitskriterien erfüllen und dabei auf verfahrenstechnisch möglichst einfachem Wege in hohen Ausbeuten herstellbar sind.

Zur Herstellung von Kohlensäureestern sind zahlreiche Methoden bekannt [Ullmann's Encyclopedia of Industrial Chemistry, Band 5, 5. Auflage, VCH Verlagsgesellschaft, Weinheim 1986, S. 197 ff und zit. Lit., Kirk-Othmer, Encyclopedia of Chemical Technology, Vol. 4, John Wiley and Sons, New York N.Y. 1978, S. 766 ff. und zit. Lit.], von denen an dieser Stelle lediglich die Umsetzung von Phosgen mit Alkohlen oder die basenkatalysierte Umesterung von geeigneten Kohlensäureestern exemplarisch genannt sein sollen.

Daneben stellt die Umesterung von Diethylcarbonat mit Propan-1,3-diol in Gegenwart von Natrium- bzw. Natriummethylat zu Trimethylencarbonat eines der ältesten Herstellungsverfahren dar [W.H. Carothers und F.v. Natta, J. Am. Chem. Soc. 52 (1930) 322; S.S. Sarel, L.A. Pohoryles und R. Ben-Shoshan, J. Org. Chem. 24 (1959) 1873], jedoch ist die Reinheit des dabei erhaltenen Produktes im Hinblick auf dessen Einsatz in Polymerisationsreaktionen bei weitem nicht ausreichend und führt zu einem qualitativ minderwertigen Produkt. Daneben läßt die unbefriedigende Ausbeute eine derartige Umesterungsreaktion für einen industriellen Einsatz als ungeeignet erscheinen.

Es ist die Aufgabe der vorliegenden Erfindung, ein Verfahren zur Verfügung zu stellen, welches die Herstellung von Kohlensäureestern auf industriell anwendbare Weise und in guten Ausbeuten erlaubt.

Es ist eine weitere Aufgabe der Erfindung, ein Verfahren zur Herstellung von cyclischen Kohlensäureestern - insbesondere von 1,3-Dioxan-2-on (Trimethylencarbonat) - zur Verfügung zu stellen, das einen Kohlensäureester liefert, der eine Reinheit aufweist, die den Einsatz des cyclischen Carbonats zur Herstellung hochmolekularer Polyester erlaubt.

Erfindungsgemäß wird die Aufgabe dadurch gelöst, daß man ein offenkettiges Dialkylcarbonat der allgemein Formel II, worin R′ und R˝ unabhängig voneinander Methyl, Ethyl, Propyl oder Phenyl bedeuten können - vorzugsweise Kohlensäurediethylester - mit einem Diol der allgemeinen Formell III, worin A die eingangs angegebene Bedeutung besitzt, vozugsweise mit 1,3-Propandiol - in Gegenwart von Zinkpulver, Zinkoxyd, Zinnpulver, einem Zinnhalogenid - wie z. B. SnCl₂ oder SnBr₂ - oder einem Zinnorganyl bei erhöhter Temperatur im Bereich von 120 - 180°C - vorzugsweise im Bereich von 140 - 150°C umsetzt. In Abhängigkeit von der Reaktivität des Kohlensäureesters und des Katalysators kann auch die Wahl einer höheren bzw. niedrigeren Reaktionstemperatur notwendig sein. Als Katalysatoren dienen neben den oben genannten Elementen bzw. Verbindungen Zinnverbindungen der allgemeinen Struktur 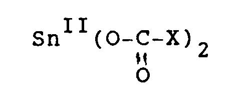
worin X einen verzweigten oder unverzweigten Alkyl-, Hydroxyalkyl- oder Alkenylrest mit bis zu 19 Kohlenstoffatomen oder einen Naphthylrest bedeutet, oder Verbindungen der allgemeinen Struktur 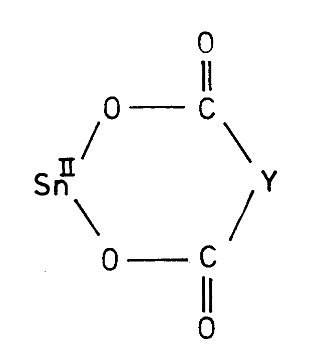
worin Y einen verzweigten oder unverzweigten Alkylen-, Hydroxyalkylen- oder Alkenylenrest mit bis zu 18 Kohlenstoffatomen oder einen Phenylrest bedeutet.

Geeignete Alkyl- bzw. Alkylengruppen für X und Y sind beispielsweise der Methyl-, Ethyl-, n- oder iso-Propyl, n-, sec.- oder tert.-Butyl, Pentyl-, Hexyl-, Heptyl- oder Octylrest, der gegebenenfalls eine oder mehrere Hydroxygruppen enthalten kann. Entsprechende Alkenyl- bzw. Alkenylenreste enthalten eine oder mehrere Doppelbindungen.

Bevorzugte Katalysatoren sind Zinnlactat, Zinntartrat, Zinnoxalat, Zinndicaprylat, Zinndilaurat, Zinndipalmitat, Zinndistearat, das Zinndioleat (Derivat der Ölsäure), Zinn-α-naphthoeat oder Zinn-β-naphthoeat und Zinndioctoat - auch als Zinn-di-(2-ethylhexanoat) bezeichnet - oder Zinn- oder Zinkpulver, worunter neben Zinn- und Zinkpulver Zinn-di-(2-ethylhexanoat) besonders bevorzugt wird.

Anschließend werden bei erhöhter Temperatur, die in einem Temperaturintervall von 80°C bis 150°C und vorzugsweise in einem Bereich von 80 bis 130°C liegt und - gegebenenfalls unter vermindertem Druck - die flüchtigen Nebenprodukte bzw. unumgesetzten Edukte abdestilliert.

Auch hierbei kann die Wahl einer höheren oder niederen Temperatur bzw. die Wahl eines niedrigen Druckes in Abhängigkeit von den Edukten bzw. den Nebenprodukten erforderlich sein.

Der Destillationsrückstand wird anschließend unter vermindertem Druck - bevorzugterweise im Feinvakuum
- fraktioniert destilliert und - falls erforderlich
- umkristallisiert.

Die nachfolgenden Beispiele sollen die Erfindung erläutern, ohne sie einzuschränken.

### Beispiel 1

761 g 1,3-Propandiol, 1477 g Diethylcarbonat und 50 g Zinnpulver werden nacheinander in einen 2,5 l-Sulfierkolben gegeben. Bei einer Ölbadtemperatur von 150°C wird anschließend über einen Dephlegmator (80°C) unter Rühren abdestilliert. Nach beendeter Destillation wird der Dephlegmator entfernt, die Badtemperatur auf 130°C abgesenkt und bei leichtem Vakuum weiterdestilliert. Der viskose Destillationsrückstand wird in einen 2 l-Kolben überführt und im Feinvakuum fraktioniert (Badtemperatur bis 180°C). Hierbei wird eine Hauptfraktion von 923 g (= 90 % d. Th.) erhalten. Umkristallisation dieser Fraktion aus Aceton/Diethylether liefert 803 g (= 79 % d. Th.) gereinigtes Produkt.

### Polymerisationsbeispiel

3.0 g des in Beispiel 1 erhaltenen und gereinigten Trimethylencarbonats werden in einem unter Stickstoff abgeschmolzenen Glasröhrchen unter Zusatz von 1-Dodecanol und SnCl₂ x2 H₂O bei 190°C polymerisiert. Der Umsetzungsgrad der Polymerisation beträgt 97 % (¹H-NMR, CDCl₃, 250 MHz). Die inhärente Viskosität des Polymeren (gemessen in 0,1 %-iger Lösung in CHCl₃ bei 25°C) beträgt 1.22 dl/g.

### Beispiel 2

380,5 g 1,3-Propandiol, 649,7 g Diethylcarbonat und 30 g Zinkstaub werden in einem 1,5 l Sulfierkolben zusammengegeben. Bei einer Badtemperatur von 140°C-175°C wird über eine Füllkörperkolonne das bei der Reaktion entstehende Ethanol abdestilliert, wobei die Reaktionsmischung intensiv gerührt wird.

Nach beendeter Reaktion wird das erhaltene Reaktionsgemisch im Vakuum (0,3-0,5 mbar) fraktioniert destilliert. Man erhält eine Hauptfraktion von 450 g, die als Schmelze in Methyl-tert.-butylether ausgefällt wird. Das Produkt wird nach dem Absaugen im Vakuumtrockenschrank bei 20°C getrocknet.
- Ausbeute:: 401,7 g = 78,7 % d. Th.

Das aus diesem Produkt hergestellte Polytrimethylencarbonat (vgl. oben stehendes Polymerisationsbeispiel) hat eine inhärente Viskosität von 1,5 dl/g (gemessen in 0,1 %-iger Lösung in CHCl₃ bis 25°C).

### Beispiel 3

401,7 g des nach Beispiel 2 erhaltenen Trimethylencarbonats werden aufgeschmolzen und in Benzin (Fraktion 40°C-60°C) ausgefällt. Nach Isolierung und Trocknung beträgt die inhärente Viskosität des daraus hergestellten Polymeren (siehe Polymerisationsbeispiel) 1,75 dl/g.
- Ausbeute:: 395,7 g = 98 % d. Th.

### Beispiel 4

401,7 g des nach Beispiel 2 erhaltenen Trimethylencarbonats werden aus getrocknetem Toluol umkristallisiert. Nach Isolierung und Trocknung hat ein daraus hergestelltes Poly-Trimethylencarbonat (siehe Polymerisationsbeispiel) eine inhärente Viskosität von 1,98 dl/g.
- Ausbeute:: 364,3 g = 90,7 % d. Th.

## Patentansprüche

1. Verfahren zur Herstellung von cyclischen Kohlensäureestern der allgemeinen Formel (I) 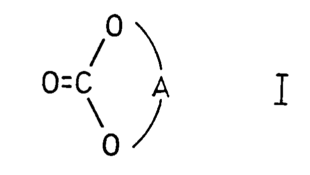 worin
A die Bedeutung einer gegebenenfalls substituierten Alkylen-Brücke des Typs
-CR¹R²-CH₂-, -CHR¹-CHR²-,
-CR¹R²-(CH₂)₂-, -CHR¹-CHR²-CH₂-,
-CH₂-CR¹R²-CH₂-, -CHR¹-CH₂-CHR²-,
und
R¹ und R² gleich oder verschieden sein können und Wasserstoff, Methyl, Ethyl, Propyl, Isopropyl, n-Butyl, sec-Butyl, tert.-Butyl, Allyl, But-2-enyl, But-3-enyl, Pent-2-enyl, Pent-3-enyl, Pent-4-enyl, Phenyl oder Benzyl bedeuten können,
dadurch gekennzeichnet, daß man einen Kohlensäureester der allgemeinen Formel II 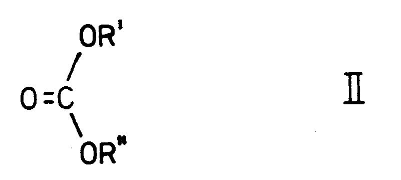 worin
R' und R'' unabhängig voneinander Methyl, Ethyl, Propyl und Phenyl bedeuten können, mit einem Diol der allgemeinen Formel III 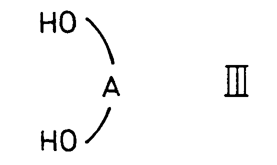 worin A und die Reste R¹ und R² die oben angegebene Bedeutung haben, bei erhöhter Temperatur in Gegenwart von Zinn oder Zink oder deren Verbindungen bei erhöter Temperatur umsetzt und das Reaktionsprodukt isoliert.

2. Verfahren nach Anspruch 1, dadurch gekennzeichnet, daß als Katalysator Zinkpulver, Zinkoxyd, Zinnpulver oder ein Zinnhalogenid eingesetzt wird

3. Verfahren nach Anspruch 1, dadurch gekennzeichnet, daS als Katalysator eine Verbindung der allgemeinen Formel 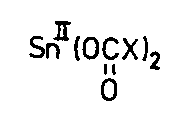 worin
X einen verzweigten oder unverzweigten Alkyl-, Hydroxyalkyl- oder Alkenylrest mit bis zu 19 Kohlenstoffatomen oder einen Naphthylrest bedeuten kann
oder eine Verbindung der allgemeinen Formel 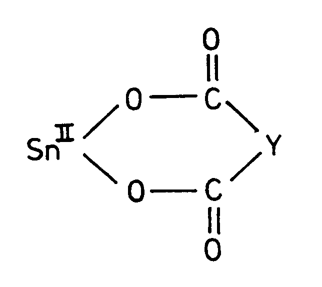 worin
Y einen verzweigten oder unverzweigten Alkylen-, Hydroxyalkylen- oder Alkenylenrest mit bis zu 18 Kohlenstoffatomen oder einen Phenylrest bedeuten kann.

4. Verfahren nach Anspruch 1, dadurch gekennzeichnet, daß als Katalysator ein Zinnorganyl eingesetzt wird.

5. Verfahren nach den Ansprüchen 1 bis 4, dadurch gekennzeichnet, daß die Reaktionstemperatur in einem Intervall von 120 bis 180°C liegt.

6. Verfahren nach den Ansprüchen 1 bis 4, dadurch gekennzeichnet, daß die Reaktionstemperatur in einem Intervall von 140 bis 150°C liegt.

7. Verfahren nach den Ansprüchen 1 bis 6, dadurch gekennzeichnet, daß man als Kohlensäureester Diethylcarbonat und als Diol 1,3-Propandiol einsetzt.

8. Verfahren nach Anspruch 7, dadurch gekennzeichent, daß man als Katalysator Zinnpulver einsetzt.

9. Verfahren nach Anspruch 7, dadurch gekennzeichnet, daß man als Katalysator Zinkpulver einsetzt

10. Verfahren nach den Anspruch 1 bis 7, dadurch gekennzeichnet, daß man nach erfolgter Umsetzung flüchtige Reaktionsprodukte aus der Reaktionsmischung destillativ abtrennt.

11. Verfahren nach den Anspruch 1 bis 7, dadurch gekennzeichnet, daß man den resultierenden cyclischen Kohlensäureester durch fraktionierte Destillation reinigt.

## Claims

1. A process for preparing cyclic carbonic acid esters of general formula I 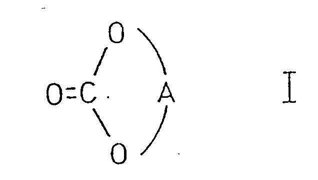 wherein
A represents an optionally substituted alkylene bridge of the type
-CR¹R²-CH₂-, -CHR¹-CHR²-,
-CR¹R²-(CH₂)₂-, - CHR¹-CHR²-CH₂-,
-CH₂-CR¹R²-CH₂-, -CHR¹-CH₂-CHR²-,
and
R¹ and R² may be the same or different and may denote hydrogen, methyl, ethyl, propyl, isopropyl, n-butyl, sec-butyl, tert.-butyl, allyl, but-2-enyl, but-3-enyl, pent-2-enyl, pent-3-enyl, pent-4-enyl, phenyl or benzyl,
characterised in that a carbonic acid ester of general formula II 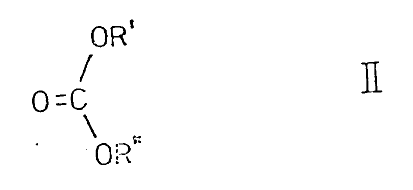 wherein
R' and R'' independently of each other may represent methyl, ethyl, propyl and phenyl, is reacted with a diol of general formula III 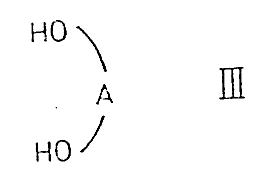 wherein A and the groups R¹ and R² are as defined hereinbefore, at elevated temperature in the presence of tin or zinc or compounds thereof, and the reaction product is isolated.

2. A process according to claim 1, characterised in that zinc powder, zinc oxide, tin powder or a tin halide is used as catalyst.

3. A process according to claim 1, characterised in that the catalyst used is a compound of general formula 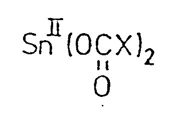 wherein
X denotes a branched or unbranched alkyl, hydroxyalkyl, or alkenyl group with up to 19 carbon atoms, or a napthyl group or a compound of general formula 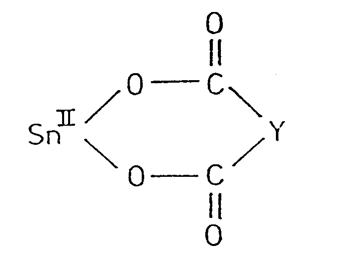 wherein
Y may represent a branched or unbranched alkylene, hydroxyalkylene or alkenylene group with up to 18 carbon atoms, or a phenyl group.

4. A process according to claim 1, characterised in that an organo-tin compound is used as catalyst.

5. A process according to claims 1 to 4, characterised in that the reaction temperature is in the range from 120 - 180°C.

6. A process according to claims 1 to 4, characterised in that the reaction temperature is in the range from 140° - 150°C.

7. A process according to claims 1 to 6, characterised in that diethylcarbonate is used as the carbonic acid ester and 1,3-propanediol is used as the diol.

8. A process according to claim 7, characterised in that tin powder is used as catalyst.

9. A process according to claim 7, characterised in that zinc powder is used as catalyst.

10. A process according to claims 1 to 7, characterised in that, after the reaction has ended, volatile reaction products are removed from the reaction mixture by distillation.

11. A process according to claims 1 to 7, characterised in that the resulting cyclic carbonic acid ester is purified by fractional distillation.

## Revendications

1. Procédé de préparation de carbonates cycliques de formule générale (I) 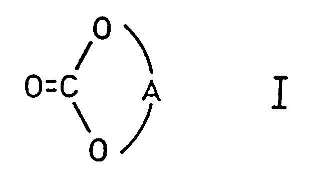 dans laquelle
A peut avoir la signification d'un pont alkylène éventuellement substitué du type
-CR¹R²-CH₂-, -CHR¹-CHR²-,
-CR¹R²-(CH₂)₂-, -CHR¹-CHR²-CH₂-,
-CH₂-CR¹R²-CH₂-, -CHR¹-CH₂-CHR²-,
et
R¹ et R² peuvent être identiques ou différents et peuvent signifier l'hydrogène, un groupe méthyle, éthyle, propyle, isopropyle, n-butyle, sec-butyle, tert.-butyle, allyle, but-2-ényle, but-3-ényle, pent-2-ényle, pent-3-ényle, pent-4-ényle, phényle ou benzyle,
caractérisé en ce que l'on fait réagir un carbonate de formule générale II 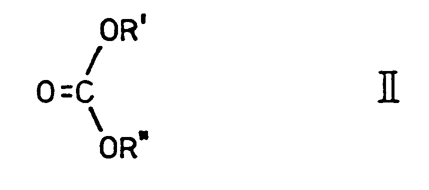 dans laquelle
R' et R'' peuvent signifier indépendamment l'un de l'autre un groupe méthyle, éthyle, propyle ou phényle, avec un diol de formule générale III 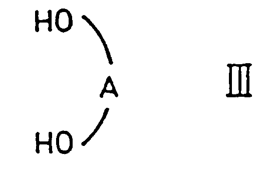 dans laquelle A et les restes R¹ et R² ont la signification indiquée ci-dessus, à haute température en présence d'étain ou de zinc ou de leurs composés et on isole le produit de la réaction.

2. Procédé selon la revendication 1, caractérisé en ce que l'on utilise comme catalyseur de la poudre de zinc, de l'oxyde de zinc, de la poudre d'étain ou un halogénure d'étain.

3. Procédé selon la revendication 1, caractérisé en ce que l'on utilise comme catalyseur un composé de formule générale 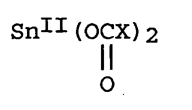 dans laquelle
X peut signifier un reste alkyle, hydroxyalkyle ou alcényle ramifié ou non ramifié ayant jusqu'à 19 atomes de carbone ou un reste naphtyle
ou un composé de formule générale 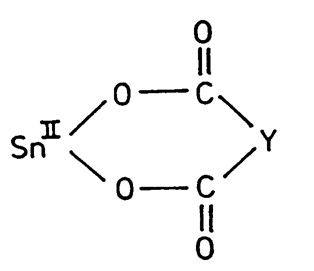 dans laquelle
Y peut signifier un reste alkylène, hydroxyalkylène ou alcénylène ramifié ou non ramifié ayant jusqu'à 18 atomes de carbone ou un reste phényle.

4. Procédé selon la revendication 1, caractérisé en ce que l'on utilise comme catalyseur un organylétain.

5. Procédé selon les revendications 1 à 4, caractérisé en ce que la température de réaction est située dans un intervalle de 120 à 180°C.

6. Procédé selon les revendications 1 à 4, caractérisé en ce que la température de réaction est située dans un intervalle de 140 à 150°C.

7. Procédé selon les revendications 1 à 6, caractérisé en ce que l'on utilise comme carbonate le carbonate de diéthyle et comme diol le propane-1,3-diol.

8. Procédé selon la revendication 7, caractérisé en ce que l'on utilise comme catalyseur de la poudre d'étain.

9. Procédé selon la revendication 7, caractérisé en ce que l'on utilise comme catalyseur de la poudre de zinc.

10. Procédé selon les revendications 1 à 7, caractérisé en ce que l'on sépare du mélange réactionnel les produits de réaction volatils par distillation après la réaction.

11. Procédé selon les revendications 1 à 7, caractérisé en ce que l'on purifie par distillation fractionnée le carbonate cyclique résultant.
